# EUROPÄISCHE PATENTSCHRIFT

(11) **EP 3 435 900 B1**
(45) Veröffentlichungstag und Bekanntmachung des Hinweises auf die Patenterteilung: **13.05.2020**
(21) Anmeldenummer: 17725500.7
(22) Anmeldetag: 27.03.2017
(51) Int. Cl.: A61B 17/72, A61F 2/36

(54) **IMPLANTIERBARE AUSGLEICHSMANSCHETTE FÜR EINE ENDOPROTHESE**
IMPLANTABLE COMPENSATING SLEEVE FOR AN ENDOPROSTHESIS
MANCHON ADAPTATEUR IMPLANTABLE POUR ENDOPROTHÈSE

(30) Priorität: 29.03.2016 DE 102016003837
(43) Veröffentlichungstag der Anmeldung: 06.02.2019
(73) Patentinhaber: Merete Holding GmbH, 10719 Berlin (DE)
(72) Erfinder: ANAPLIOTIS, Emmanuel, 14195 Berlin (DE)
(74) Vertreter: Bittner, Thomas L.
(86) Internationale Anmeldenummer: PCT/DE2017/000084
(87) Internationale Veröffentlichungsnummer: WO 2017/167324

(56) Entgegenhaltungen:
- EP-A1- 0 761 175
- JP-B2- 2 955 701
- US-A- 3 977 398

## Beschreibung

Die Erfindung betrifft eine implantierbare Ausgleichsmanschette für eine Endoprothese, insbesondere für eine liegende Endoprothese, zur intramedullären Versorgung einer periprothetischen oder interprothetischen Fraktur.

Aufgrund der hohen Anzahl implantierter Hüft- und Knieprothesen bei steigendem Alter der operierten Patienten nimmt die Häufigkeit periprothetischer oder interprothetischer Frakturen zu.

Periprothetische Frakturen sind Brüche in der Umgebung einer Prothese und treten oftmals in Folge eines Sturzes auf. Auch kann eine schlechte Knochenqualität aufgrund von osteoporotischen oder osteolytischen Veränderungen zu einer periprothetischen Fraktur führen.

Interprothetische Frakturen treten zwischen zwei im selben Knochen einliegenden Implantaten bzw. Prothesen auf. Am häufigsten findet man interprothetische Frakturen im Femur zwischen einer Hüft- und einer Knieprothese.

In Abhängigkeit von Art und Verlauf der Fraktur, deren Ort in Bezug auf die Prothese sowie in Abhängigkeit von der Sitzstabilität der Prothese sind unterschiedlichste Therapieformen indiziert.

Insbesondere ist für die Wahl der Frakturversorgung entscheidend, ob sich etwaig der Sitz der Prothese gelockert hat. Insofern die Prothese weiterhin fest im Knochen verankert ist, kann größtenteils von einer Revision der Prothese abgesehen werden.

Die Standardmethode bei der Versorgung von peri- oder interprothetischen Frakturen, die als Typ B1 gemäß der Vancouver-Klassifikation kategorisiert werden, stellt die winkelstabile Plattenosteosynthese dar. Oftmals führt aber gerade die Versorgung mittels Plattenosteosynthese zu einer Schwächung des prothetisch versorgten Knochens unter Bildung von Sollbruchstellen, in deren Bereich es häufig zu Folgebrüchen kommt. Je nach Befundslage kann dann der Einsatz einer Megaprothese als vollständiger Femurersatz unumgänglich sein.

Insofern die Knochenstruktur eine Osteosynthese nicht zulässt, stellt die intramedulläre Stabilisierung eine alternative Operationstechnik dar, bei der der Defekt mittels eines individuell gefertigten Interpositionsnagels überbrückt wird. Interpositionsnägel bieten eine hohe Stabilität und werden in unterschiedlichen Größen bereitgestellt.

In den Fällen von Frakturen des Typs Vancouver B1, bei denen trotz der Fraktur die Hüft- oder Knieprothese weiterhin fest im Knochen verankert ist, kann auf eine weitere Versorgungsmethode zurückgegriffen werden, bei der die implantierte Prothese im Körper verbleiben kann. Bei dieser Operationstechnik wird z.B. zur Überbrückung des Knochendefektes im Femur der Schaft einer implantierten Hüftprothese mit einem Femurnagel, der distal in den Markkanal eingesetzt wird, über ein manschettenartiges Zwischenmodul verbunden, wodurch der Schaft mechanisch verlängert werden kann. Diese Methodik bietet viele Vorteile. Einerseits kann die Dauer und Tiefe des chirurgischen Eingriffs deutlich gemindert, anderseits kann mit einer verbesserte Heilung und eine höhere Stabilität des prothetisch versorgten Knochens bei gleichzeitig reduzierter Komplikationsrate gerechnet werden.

Es existieren jedoch nur einige wenige modulare Endoprothesesysteme, bei denen die Implantat- bzw. Prothese-Komponenten derart aufeinander abgestimmt, dass eine bereits implantierte Prothese in Folgeoperationen stabil endoprothetisch erweitert werden kann.

Oftmals kann sowohl die Herkunft eines Protheseschaftes als auch dessen Geometrie und Beschaffenheit erst eindeutig festgestellt werden, wenn dieser operativ freigelegt wurde. Der Chirurg steht dann vor einer schnell zu treffenden Entscheidung, ob ein bereitgestelltes Endoprothesesystem mit dem vorgefundenen Schaft kompatibel wäre und, ob das nachträglich implantierte Endoprothesesystem mit dem Schaftende rotationsstabil verbunden werden kann.

Der Medizinproduktemarkt bietet unzählige Hüft- und Knieprothesen an, die sich in deren Konfiguration und Ausmaßen deutlich voneinander unterscheiden. Die wesentlichen Unterschiede finden sich insbesondere in der Länge und im Querschnittsprofil der Protheseschäfte sowie in deren Oberflächenbeschaffenheiten. Einige Prothesen weisen u.a. Riffelungen oder in Längsrichtung verlaufende Rillenprofile auf, die eine intramedulläre Erweiterung zu einer Endoprothese aufgrund einer nur unzufrieden herstellbaren Kraftschlüssigkeit zwischen den Protheseteilen erschweren.

Aus dem Stand der Technik sind endoprothetische Verbindungssysteme-Systeme bekannt, mit denen freiliegende Schaftenden nach einer periprothetischen Fraktur endoprothetisch verlängert werden können.

Die DE 10 2008 062 226 A1 offenbart eine Verlängerung eines proximalen Femur-Nagels. Die Verlängerung in Form eines retrograd in den in den Markraum des Femurs einbringbaren distalen Femur-Nagels weist am proximalen Ende eine Aufnahmeöffnung auf, die über das distale Ende des proximalen Femur-Nagels geschoben wird. Die Sicherung erfolgt über eine Verriegelungsschraube, die zusätzlich mit dem Femur verbunden ist.

Die US 8 668 692 B1 beschreibt ein periprothetisches Endoprothese-System für einen bereits implantierten Protheseschaftes. Dieses Endoprothese-System umfasst ein Verbindungsstück, das einen in Längsrichtung verlaufenden und dreiteilig ausgebildeten Kanal aufweist. Der proximale Bereich dieses Kanals ist für die Aufnahme des innenliegenden Schaftendes hergerichtet und ist zur Anpassung an dessen Schaftform konisch ausgebildet. Die Verbindung zwischen dem innenliegendem Schaftende und dem konisch geformten Kanalbereich erfolgt über Kraftschluss, mit oder ohne zusätzliche Einzementierung des Schaftendes. In den distalen Endbereich des Kanals kann zur endoprothetischen Verlängerung ein weiteres Implantat eingeschraubt werden.

Des Weiteren wird auf die Offenbarung der DE 39 09 182 C1 als einschlägigen Stand der Technik verwiesen.

Aufgabe der Erfindung ist es, ein implantierbares Ergänzungsbauteil für eine endoprothetische Verlängerung eines innenliegenden Implantats anzugeben, mit welchem zur Vermeidung einer Revision des innenliegenden Implantats der Kraftschluss zwischen dem innenliegenden Implantat und einer endoprothetischen Verlängerung derart verbessert werden kann, dass eine rotationsstabile Verbindung zwischen dem innenliegenden Implantat und der endoprothetischen Verlängerung erzielt werden kann.

Die Aufgabe wird mit einer implantierbaren Ausgleichsmanschette gemäß Anspruch 1 für eine Endoprothese gelöst, die zur Anbringung zwischen einem länglichen Implantatabschnitt eines ersten Implantats und einem den länglichen Implantatabschnitt des ersten Implantats umgreifenden zweiten Implantat hergerichtet ist. Die Ausgleichsmanschette ist als Hülse ausgebildet, die einen Hülsenkörper und eine vom proximalen zum distalen Ende des Hülsenkörpers durchgehenden Durchführung zur Aufnahme des länglichen Implantatabschnitts des ersten Implantats aufweist. Der Hülsenkörper ist durch in Längsrichtung in den Hülsenkörper einschneidende Schlitze in mäanderförmige Segmente mit jeweils zwei Längsarmen geteilt, wobei die Schlitze alternierend vom distalen und vom proximalen Rand des Hülsenkörpers ausgehen. Der Hülsenkörper ist mindestens in einem Teilabschnitt radial dehnbar ausgestaltet. Die Ausgleichsmanschette zeichnet sich dadurch aus, dass auf der der Durchführung zugewandten Innenfläche des Hülsenkörpers unter Ausbildung eines in die Durchführung hineinragenden Innenprofils mindestens eine in Längsrichtung eines mäanderförmigen Segmentes verlaufende Aufwölbung eingerichtet ist.

Die erfindungsgemäße Ausgleichsmanschette zeichnet sich dadurch aus, dass diese aufgrund deren Verformbarkeit flexibel an eine vorgefundene Querschnittsform eines Schaftes oder Nagel als erstes innenliegendes Implantat anpassbar ist. Durch die innenseitig in Längsrichtung verlaufenden Aufwölbungen kann ein vorhandenes Rillenprofil ausgeglichen und dadurch eine vergrößerte Kontaktfläche für eine rotationsstabile Endoprotheseerweiterung gebildet werden.

Der Begriff "erstes Implantat" bezeichnet insbesondere Hüftprothesen, Knieprothesen oder Schulterprothese sowie Femur-, Tibia- und Gammanägel oder allgemein in das Knochenmark eingebrachte Knochennägel. Insbesondere bezeichnet der Begriff "erstes Implantat" innenliegende Implantate.

Der Begriff "länglicher Implantatabschnitt" bezeichnet einerseits einen Protheseschaft, im Speziellen den im Knochen verankerten Endbereich eines Protheseschaftes, andererseits aber auch einen Nagelendbereich eines Knochennagels.

Mit dem Begriff "zweites Implantat" ist ein Implantat bezeichnet, das dazu hergerichtet ist, mit dem länglichen Implantatabschnitt des ersten Implantats verbunden zu werden, indem der längliche Implantatabschnitt des ersten Implantats in eine hülsenförmige Aufnahmeeinrichtung im zweiten Implantat eingeführt und in dieser mittels Kraftschluss fixiert wird.

Die baulichen Unterschiede zwischen dem ersten Implantat und dem zweiten Implantat können durch Anbringen der erfindungsgemäßen Ausgleichsmanschette auf dem Endabschnitt des ersten Implantats ausgeglichen werden, wodurch es für den Chirurgen möglich wird, entweder überhaupt erst eine Koppelung von erstem und zweitem Implantat zu erzielen und/oder die Sitzfestigkeit des Verbundes zwischen beiden Implantaten derart deutlich zu steigern, dass die geforderte Rotationsstabilität erreicht wird.

Die zur Ausbildung der Mäanderform in den Hülsenkörper eingebrachten Schlitze gehen abwechselnd vom distalen Rand und vom proximalen Rand aus und verlaufen gegenläufig linear in Längsrichtung des Hülsenkörpers. Die Schlitze reichen dabei vorteilhafterweise bis in den Randbereich des gegenüberliegenden Randes hinein, wodurch im proximalen Randbereich erste Querstege und im distalen Randbereich zweite Querstege ausgebildet werden. Nur zur Definitionszwecken wird festgelegt, dass die ersten Querstege zwei benachbarte Längsarme zu einer Längsarmpaarung eines Mäandersegments und die zweiten Querstege zwei benachbarte Mäandersegmente miteinander verbinden.

In einer vorteilhaften Ausgestaltungsform sind die Längsarme eines Mäandersegmentes oder aller Mäandersegmente parallel angeordnet. In einer anderen Ausgestaltungsform können die Schlitze in Zick-Zack-Form oder in Kurvenform ausgebildet sein.

In einer weiteren Ausführungsform entspricht die Breite eines ersten und/oder zweiten Querstegs der Breite eines anschließenden Längsarmes. Vorteilhafterweise weisen alle Längsarme die gleiche Breite auf.

Durch die Aufsplittung des Hülsenkörpers in mäanderförmige Segmente wird mindestens in einem Teilbereich des Hülsenkörpers eine Dehnbarkeit in radialer Richtung erreicht, wodurch die Hülse aufgeweitet und unter Klemmwirkung auf einen länglichen Implantatabschnitt eines ersten Implantats aufgeschoben werden kann.

Durch die Formflexibilität des in Mäandersegmente unterteilten Hülsenkörpers kann sich die Ausgleichsmanschette beim Aufschieben auf den länglichen Implantatabschnitt eines ersten Implantats an nahezu jede Querschnittsform und Querschnittsverläufe des länglichen Implantatabschnitts anpassen, so dass diese mit unterschiedlich geformte Schaftspitzen oder Nagelenden kompatibel ist.

Die innenseitig des Hülsenkörpers in Längsrichtung eines mäanderförmigen Segmentes verlaufende Aufwölbung ist zum Eingriff in eine in Längsrichtung des länglichen Implantatabschnitt eines ersten Implantats verlaufende Rille hergerichtet. Durch den Eingriff einer Aufwölbung in eine Längsrille im ersten Implantat wird eine unerwünschte Verdrehung von länglichem Implantatabschnitt und der darauf aufgeschobenen Ausgleichsmanschette verhindert.

In einer vorteilhaften Ausgestaltung erstreckt sich die mindestens eine in Längsrichtung eines mäanderförmigen Segmentes verlaufende Aufwölbung über die gesamte Breite zweier benachbarter Längsarme sowie übe den zwischen diesen beiden Längsarmen angeordneten Schlitz eines mäanderförmigen Segments. In dieser Ausführungsform ist die Aufwölbung somit in Längsrichtung geteilt ausgebildet, vorteilhafterweise hälftig geteilt ausgebildet.

Durch diese Ausgestaltungsform kann die in eine Längsrille des ersten Implantats eingreifende Aufwölbung gespreizt werden, so dass durch das Auseinanderspreizen der die Aufwölbung tragenden Längsarme diese gegenläufig in seitlicher Richtung gegen die Rillenwand gedrückt werden, wodurch neben dem Klemmsitz der Ausgleichsmanschette eine zweite Klemmkraft innerhalb einer Rille erzeugt und dadurch die Sitzstabilität der Ausgleichsmanschette erhöht wird. Für eine Optimierung der Sitzfestigkeit kann das Innenprofil der Ausgleichsmanschette komplementär zu dem Rillenprofil im länglichen Implantatabschnitt des ersten Implantats ausgestaltet sein.

In einer weiteren Ausgestaltungsform bilden die auf der der Durchführung zugewandten Innenfläche des Hülsenkörpers eingerichteten Aufwölbungen ein umlaufendes und im Querschnitt der Ausgleichsmanschette wellenförmiges Innenprofil mit Wellenbergen und Wellentälern aus. Insbesondere erstrecken sich bei einem solchen Innenprofil ein Wellenberg und/oder ein Wellental des wellenförmigen Innenprofils jeweils über zwei benachbarte Längsarme eines mäanderförmigen Segments, bevorzugt ist das wellenförmige Innenprofil achssymmetrisch zur Längsachse der Ausgleichsmanschette ausgestaltet.

Für eine Verstärkung der Klemmwirkung können die in Längsrichtung des Hülsenkörpers verlaufenden Schlitze abwechselnd am Boden eines Wellentals und auf einem Wellenkamm eingerichtet sein.

Die Außenfläche der Außenmanschette bildet die für einen rotationsstabilen Kraftschluss mit dem zweiten Implantat erforderliche Kontaktfläche. Die Festigkeit des Verbunds zwischen den beiden Implantaten hängt in direkter Weise von der Größe der Kontaktfläche und zudem von der Rauheit des Materials ab. Je größer die Kontaktfläche ist, umso höher ist die Haftreibung, die einer Verdrehung der zusammengefügten Implantate entgegenwirkt.

Zur Erzeugung einer möglichst großen Kontaktfläche ist die durch die Außenfläche der Längsarme eines mäanderförmigen Segmentes gebildete Außenseite des Hülsenkörpers vorteilhafter Weise unprofiliert, d.h. oberflächlich plan, ausgebildet.

Für einen anders gearteten Fall, bei dem das zweite Implantat ebenfalls in der Aufnahme für das erste Implantat eine Innenprofilierung aufweist, kann zur Verbesserung der Sitzfestigkeit die Außenfläche der Ausgleichsmanschette mit einer zur Innenprofilierung des zweiten Implantats kompatiblen Außenprofilierung ausgestattet sein.

In einer anderweitigen Ausgestaltungsform kann die Wanddicke des Hülsenkörpers in dessen distalen Randbereich dicker als im gegenüberliegenden proximalen Randbereich des Hülsenkörpers ausgebildet sein.

Vorteilhafterweise nimmt die Wandverdickung in Längsrichtung des Hülsenkörpers gleichmäßig zu. Die in Längsrichtung zunehmende Wandverdickung kann radial umlaufend über den gesamten Hülsenkörper oder aber auch nur im Bereich eines oder mehrerer Mäandersegment des Hülsenkörpers eingerichtet sein.

Mit einer solchen erfindungsgemäßen Ausgleichsmanschette kann dann eine Passformdifferenz zwischen einem sich zur Implantatspitze hin verjüngenden ersten Implantats, beispielsweise einem sich in distaler Richtung verjüngenden Schaft einer Hüftprothese, und einer zylindrisch geformten Implantataufnahme im zweiten Implantat ausgeglichen werden.

Als Herstellungsmaterialien für eine erfindungsgemäße Ausgleichsmanschette eignen sich insbesondere chirurgische Stähle, Titan oder Titan-Legierung. Auch kann die Ausgleichsmanschette aus einem biokompatiblen Kunststoff, insbesondere aus Peek, gebildet sein.

Des Weiteren wird ein Set mit mehreren Ausgleichsmanschetten mit unterschiedlichen mittleren Innendurchmessern und/oder unterschiedlichen Innenprofilen vorgeschlagen. Zur Ermittlung des relevanten Durchmessers am länglichen Implantatabschnitt des ersten Implantats kann das Set eine Mess-Schablone umfassen.

Nachstehend wird die Erfindung anhand eines Ausführungsbeispiels näher erläutert. Die Figuren zeigen:
- Fig. 1:: Seiten- und Querschnittsansicht eines länglichen Implantatabschnitts eines ersten Implantats,
- Fig. 2:: Seitenansicht einer Ausführungsform einer erfindungsgemäßen Ausgleichsmanschette,
- Fig. 3:: Querschnittsansicht der Ausführungsform gemäß Fig. 2,
- Fig. 4:: Seitenansicht einer auf einen länglichen Implantatabschnitt gemäß Fig. 1 aufgeschobenen Ausgleichsmanschette gemäß Fig. 2 und
- Fig. 5:: Querschnittsansicht einer auf einen länglichen Implantatabschnitt gemäß Fig. 1 aufgeschobenen Ausgleichsmanschette gemäß Fig. 2.

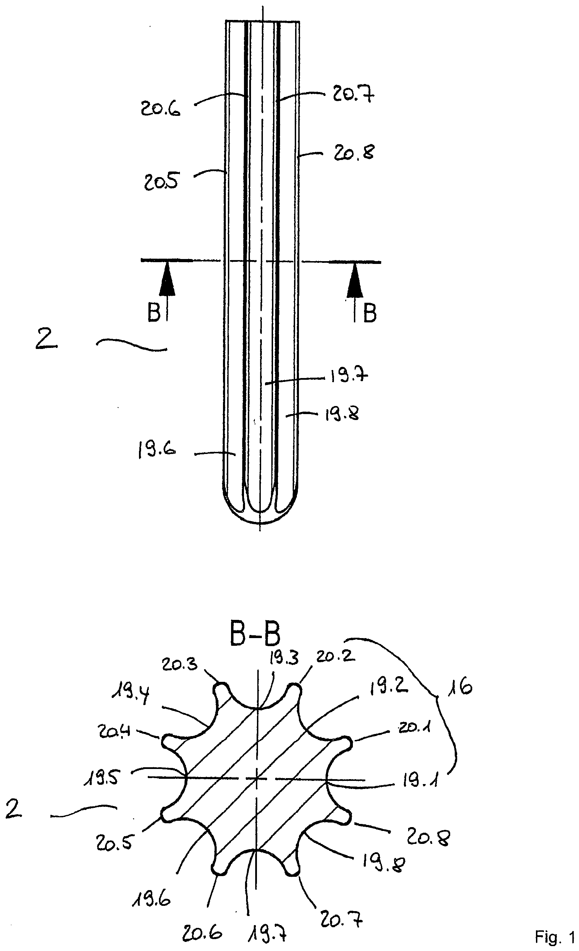

Die Figur 1 zeigt sowohl eine Seitenansicht als auch eine Querschnittsansicht B-B eines länglichen Implantatabschnitts eines ersten Implantats 2. Bei dem dargestellten ersten Implantat 2 handelt es sich um einen geradlinigen Knochennagel mit einem in Längsrichtung verlaufenden Rillenprofil 16. In der Figur 1 ist der Nagelendbereich 2 des Knochennagels abgebildet, der beispielsweise nach einer periprothetischen Fraktur operativ freigelegt wurden. Wie die Querschnittsansicht B-B zeigt, weist das wellenförmige Rillenprofil 16 im Nagelendbereich 2 des Knochennagels ausgedehnte Wellentäler 19.1 - 19.8 mit kurzen spitzen Wellenkämmen 20.1 - 20.8 auf.

Für eine kraftschlüssige Verbindung mit einem zweiten Implantat mit einer glattwandigen Aufnahmeeinrichtung, in die das Nagelendbereich 2 für eine endoprothetische Erweiterung eingeführt werden soll, steht für die Kraftübertragung daher nur eine sehr geringe Kontaktfläche zur Verfügung, da der Kontakt nur zwischen den Wellenkämmen 20.1 bis 20.8 und der Wand der Aufnahmeeinrichtung (nicht dargestellt) ausgebildet werden kann.

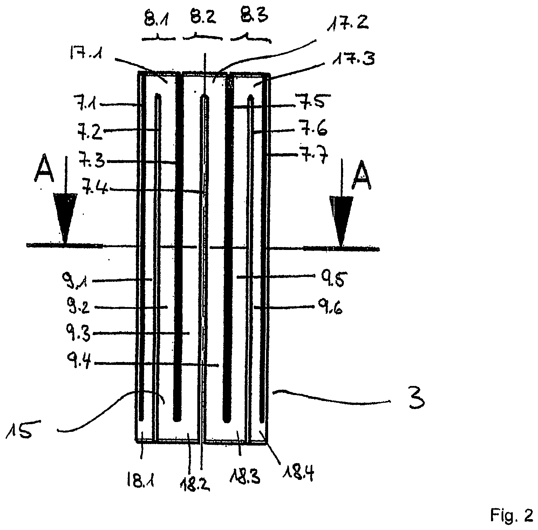

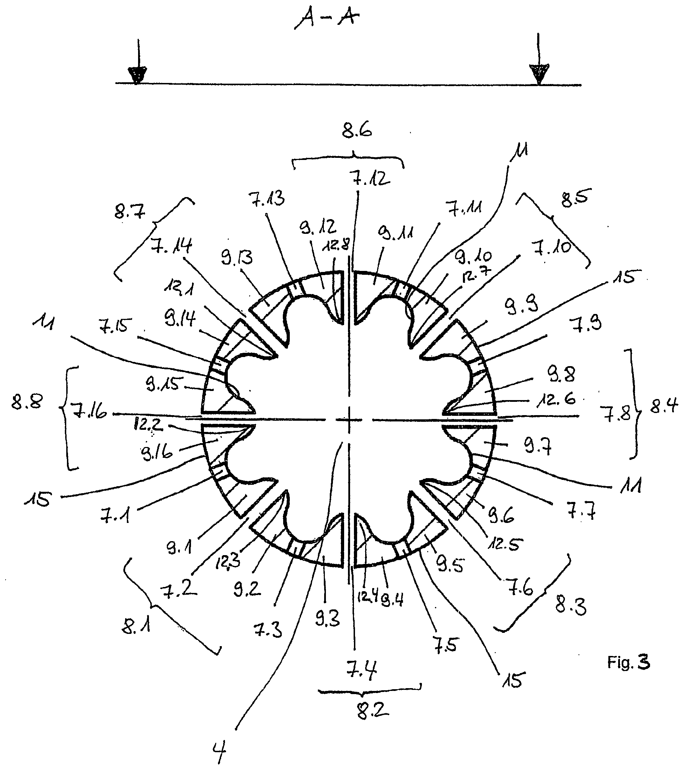

Die Figuren 2 und 3 zeigen eine Ausführungsform der erfindungsgemäßen Ausgleichsmanschette 1. Die Figur 3 gibt den Querschnitt A-A gemäß der Fig. 2 wieder.

Die Ausgleichsmanschette 1 ist als Hülse mit einem Hülsenkörper 3 und mit einer vom proximalen Ende 6 zum distalen Ende 5 des Hülsenkörpers durchgehenden Durchführung 4 zur Aufnahme des länglichen Implantatabschnitts des ersten Implantats 2 ausgebildet.

Der Hülsenkörper 3 ist durch in Längsrichtung in den Hülsenkörper 3 einschneidende und alternierend vom distalen 5 und vom proximalen Rand 6 des Hülsenkörpers 3 ausgehende Schlitze 7.1 bis 7.16 jeweils in mäanderförmige Segmente 8.1 bis 8.8 mit jeweils zwei Längsarmpaaren 9.1 - 9.2, 9.3 - 9.4, 9.5 - 9.6, 9.7 - 9.8, 9.9 - 9.10, 9.11 - 9.12, 9.13 - 9.14 und 9.15 - 9.16 geteilt. Durch die Einrichtung mäanderförmiger Segmente 8.1 bis 8.8 kann der ringförmige Hülsenkörper 3 über dessen gesamte Länge in radialer Richtung ausgedehnt bzw. aufgeweitet werden, um in pass- und formschlüssig auf das Nagelende 2 aufschieben zu können.

Die der Durchführung 4 zugewandte Innenfläche 10 des Hülsenkörpers 3 ist profiliert ausgebildet. Das Innenprofil 11 zeichnet sich durch abwechselnde Wellenberge 13.1 bis 13.8 und Wellentälern 14.1 bis 14.8 aus. Das Wellenprofil 11 wird durch auf der Innenfläche 10 des Hülsenkörpers 3 eingerichtete Aufwölbungen 12.1 bis 12.8 gebildet, die in Längsrichtung des Hülsenkörpers 3 verlaufen und sich in umlaufender Richtung jeweils über zwei Längsarmpaarungen 9.1 - 9.2, 9.3 - 9.4, 9.5 - 9.6, 9.7 - 9.8, 9.9 - 9.10, 9.11 - 9.12, 9.13 - 9.14 und 9.15 - 9.16 erstrecken.

Zudem erstreckt sich eine jede Aufwölbung 12.1 bis 12.8 über jeweils zwei benachbarte und zu einem Mäandersegment 8.1 bis 8.8 gehörende Längsarme 9.1 - 9.2, 9.3-9.4, 9.5-9.6, 9.7-9.8, 9.9-9.10, 9.11 -9.12, 9.13-9.14 und 9.15 - 9.16.

In der Gesamtbetrachtung des wellenförmigen Innenprofils 11 sind die Wellenberge 13.1 bis 3.8 und Wellentäler 14.1 bis 14.8 derart ausgestaltet, dass ein Wellenberg 13.1 -13.8 und/oder ein Wellental 14.1 - 14.8 des wellenförmigen Innenprofils 11 jeweils über die gesamte Breite einer Längsarmpaarung 9.1 - 9.2, 9.3 - 9.4, 9.5 - 9.6, 9.7 - 9.8, 9.9 - 9.10, 9.11 - 9.12, 9.13 - 9.14 und 9.15 - 9.16 eines mäanderförmigen Segments 8.1 - 8.8 verlaufen. Zudem ist das wellenförmige Innenprofil 11 achssymmetrisch zur Längsachse der Ausgleichsmanschette 1 ausgebildet.

Die in Längsrichtung des Hülsenkörpers 3 eingerichteten Schlitze 7.1 bis 7.16 verlaufen in Längsrichtung des Hülsenkörpers 3. Diese sind linear und parallel zueinander ausgerichtet sowie abwechselnd am Boden eines Wellentals 14.1 bis 14.8 sowie auf einem Wellenkamm eines Wellenbergs 13.1 bis 13.8 eingerichtet.

Die Schlitze 7.1 bis 7.16 reichen jeweils bis in den Randbereich des gegenüberliegenden Randes 5 bzw. 6 hinein, wodurch im proximalen Randbereich 6 erste Querstege 17 und im distalen Randbereich 5 zweite Querstege 18 gebildet werden, die jeweils zwei Längsarme 9 miteinander verbinden. Die ersten proximalen Querstege 16.1 bis 16.8 und die zweiten distalen Querstege 17.1 bis 17.8 sowie die jeweils daran anschließenden Längsarme 9.1 bis 9.16 weisen die gleiche Breite auf.

In der Figur 2 sind in der Frontaldarstellung des Hülsenkörpers 3 drei mäanderförmige Segmente 8.1, 8.2 und 8.3 erkennbar. Ein mäanderförmiges Segment 8.1, 8.2 bzw. 8.3 besteht jeweils aus einem ersten Quersteg 17.1, 17.2, 17.3 im proximalen Randbereich 6 und zwei daran anschließenden Längsarmpaaren 9.1-9.2, 9.3-9.4, 9.5-9.6.

Die benachbarten mäanderförmigen Segmente 8.1, 8.2 und 8.3 sind über die randseitig eingerichteten zweiten Querstege 18.1, 18.2, 18.3 und 18.4 im Hülsenkörper 3 ringförmig miteinander verbunden.

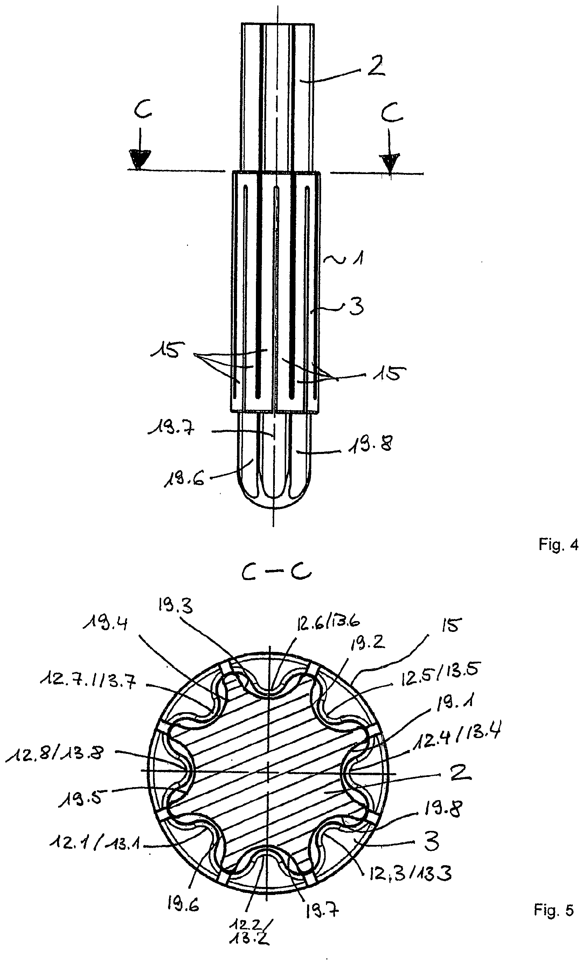

Wie aus Fig. 5 ersichtlich ist, ist das wellenförmige Innenprofil 11 komplementär zu dem Rillenprofil 16 im länglichen Implantatabschnitt des ersten Implantats 2 ausbildet.

Die Figuren 4 und 5 zeigen eine auf den Nagelendbereich eines Knochennagels 2 aufgeschobene Ausgleichsmanschette 1. Beim Aufschieben der Ausgleichsmanschette 1 wird der Hülsenkörper 3 aufgeweitet, wodurch dieser auf dem Endabschnitt 2 des Knochennagels passförmig festklemmt. Das wellenförmige Innenprofil 11 besteht aus alternierenden Wellenbergen 13.1 - 13.8 und Wellentäler 14.1 - 14.8. Jeder Wellenberg 13.1 - 13.8 bildet eine Aufwölbungen 12.1 - 12.8, die in Längsrichtung des Hülsenkörpers verläuft und jeweils in eine längsverlaufende Rille (Wellentäler 19.1 - 19.8 des Rillenprofils 16) im Knochennagel eingreift, so dass das Rillenprofil 16 durch Aufschieben der Ausgleichsmanschette 1 ausgefüllt bzw. ausgeglichen wird.

Durch die planen Außenflächen 15.1 - 15.16 der Längsarme 9.1 bis 9.16 der mäanderförmigen Segmente 8.1 bis 8.8 des Hülsenkörpers 3 wird eine insgesamt plane Außenseite 15 gebildet, die nun eine gegenüber der Kontaktfläche des Knochennagelendes 2 deutlich vergrößerte Kontaktfläche bietet, mittels derer im Kraftschluss mit einem zweiten Implantat, das den Endabschnitt des Knochennagel 2 im Bereich der angebrachten Ausgleichsmanschette 1 umgreift, eine rotationstabile endoprothetische Verlängerung des Knochennagels 2 erzielt werden kann.
- 1: Ausgleichsmanschette für eine Endoprothese
- 2: länglicher Implantatabschnitt eines ersten Implantats
- 3: Hülsenkörper
- 4: Durchführung
- 5: Distaler Randbereich
- 6: Proximaler Randbereich
- 7: Schlitz (7.1 - 7.16)
- 8: mäanderförmiges Segment (8.1 - 8.8)
- 9: Längsarm (9.1 - 9.16)
- 10: Innenfläche des Hülsenkörpers
- 11: Innenprofil
- 12: in Längsrichtung verlaufende Aufwölbung (12.1 - 12.8)
- 13: Wellenberg des Innenprofils (13.1 -13.8)
- 14: Wellental des Innenprofils (14.1 -14.8)
- 15: Außenseite des Hülsenkörpers
- 16: Rillenprofil im länglichen Implantatabschnitt des ersten Implantats
- 17: Proximaler Quersteg (17.1 - 17.8)
- 18: Distaler Quersteg (18.1 - 18.8)
- 19: Wellental des Rillenprofils 16
- 20: Wellenkamm des Rillenprofils 16

## Patentansprüche

1. Implantierbare Ausgleichsmanschette (1) für eine Endoprothese zur Anbringung zwischen einem länglichen Implantatabschnitt eines ersten Implantats (2) und einem den länglichen Implantatabschnitt des ersten Implantats umgreifenden zweiten Implantat,
wobei die Ausgleichsmanschette (1) als Hülse mit einem Hülsenkörper (3) und mit einer vom proximalen zum distalen Ende des Hülsenkörpers durchgehenden Durchführung (4) zur Aufnahme des länglichen Implantatabschnitts des ersten Implantats (2) ausgebildet ist,
der Hülsenkörper (3) durch in Längsrichtung in den Hülsenkörper (3) einschneidende und alternierend vom distalen (5) und vom proximalen Rand (6) des Hülsenkörpers ausgehende Schlitze (7.1 - 7.16) in mäanderförmige Segmente (8.1 - 8.8) mit jeweils zwei Längsarmen (9.1 - 9.16) geteilt ist, und wobei der Hülsenkörper (3) mindestens in einem Teilabschnitt radial dehnbar ist, wobei
auf der der Durchführung (4) zugewandten Innenfläche (10) des Hülsenkörpers (3) unter Ausbildung eines in die Durchführung (4) hineinragenden Innenprofils (11) mindestens eine in Längsrichtung eines mäanderförmigen Segmentes (8.1 - 8.8) verlaufende Aufwölbung (12.1 - 12.8) eingerichtet ist.

2. Implantierbare Ausgleichsmanschette nach Anspruch 1, **dadurch gekennzeichnet, dass**
sich die mindestens eine in Längsrichtung eines mäanderförmigen Segmentes (8.1 - 8.8) verlaufende Aufwölbung (12.1 - 12.8) über die zwei benachbarten Längsarme (9.1 - 9.2, 9.3 - 9.4, 9.5 - 9.6, 9.7 - 9.8, 9.9 - 9.10, 9.11 - 9.12, 9.13 - 9.14 und 9.15 - 9.16) des mäanderförmigen Segments (8.1 - 8.8) erstreckt.

3. Implantierbare Ausgleichsmanschette nach Anspruch 1 oder 2, **dadurch gekennzeichnet, dass**
auf der der Durchführung (4) zugewandten Innenfläche (2) des Hülsenkörpers (2) eingerichteten Aufwölbungen (12.1 - 12.8) ein umlaufendes und im Querschnitt der Ausgleichsmanschette wellenförmiges Innenprofil mit Wellenbergen (13.1 - 13.8) und Wellentälern (14.1 - 14.8) ausbilden.

4. Implantierbare Ausgleichsmanschette nach Anspruch 3, **dadurch gekennzeichnet, dass**
sich ein Wellenberg (13.1 - 13.8) und/oder ein Wellental (14.1 - 14.8) des wellenförmigen Innenprofils (11) jeweils über zwei benachbarte Längsarme (9.1 - 9.16) eines mäanderförmigen Segments (8) erstreckt.

5. Implantierbare Ausgleichsmanschette nach Anspruch 3 oder 4, **dadurch gekennzeichnet, dass**
die in Längsrichtung des Hülsenkörpers (3) verlaufenden Schlitze (7.1 - 7.16) abwechselnd am Boden eines Wellentals (14.1 - 14.8) und auf einem Wellenkamm eines Wellenbergs (13.1 - 13.8) eingerichtet sind.

6. Implantierbare Ausgleichsmanschette nach einem der Ansprüche 3 bis 5, **dadurch gekennzeichnet, dass**
das wellenförmige Innenprofil (11) achssymmetrisch zur Längsachse der Ausgleichsmanschette (1) ausgestaltet ist.

7. Implantierbare Ausgleichsmanschette nach einem der Ansprüche 1 bis 6, **dadurch gekennzeichnet, dass**
die durch die Außenfläche der Längsarme (7.1 - 7.16) eines mäanderförmigen Segmentes (8) gebildete Außenseite (15) des Hülsenkörpers (3) unprofiliert ausgebildet ist.

8. Implantierbare Ausgleichsmanschette nach einem der Ansprüche 1 bis 7, **dadurch gekennzeichnet, dass**
das Innenprofil (11) komplementär zu einem Rillenprofil (16) in einem länglichen Implantatabschnitts des ersten Implantats (2) ausgestaltet ist.

9. Implantierbare Ausgleichsmanschette nach einem der Ansprüche 1 bis 8, **dadurch gekennzeichnet, dass**
die Ausgleichsmanschette (1) aus einem chirurgischen Stahl, aus Titan oder aus einer Titan-Legierung gebildet ist.

10. Implantierbare Ausgleichsmanschette nach einem der Ansprüche 1 bis 8, **dadurch gekennzeichnet, dass**
die Ausgleichsmanschette (1) aus einem biokompatiblen Kunststoff, insbesondere aus PEEK gebildet ist.

## Claims

1. An implantable compensating sleeve (1) for an endoprosthesis for fitting between an elongate implant portion of a first implant (2) and a second implant that encloses the elongate implant portion of the first implant,
the compensating sleeve (1) being formed as a sheath having a sheath body (3) and having a passage (4) extending from the proximal end of the sheath body to the distal end of the sheath body for receiving the elongate implant portion of the first implant (2), the sheath body (3) being divided by slits (7.1 - 7.16) that cut into the sheath body (3) in a longitudinal direction and originate in alternation from the distal (5) and from the proximal edge (6) of the sheath body into meander-like segments (8.1 - 8.8), each meander-like segment having two longitudinal arms (9.1 - 9.16), and
the sheath body (3) being radially expandable in at least one sub-portion, wherein on the inner surface (10) of the sheath body (3) facing the passage (4), at least one bulge (12.1 - 12.8) is provided, which runs in the longitudinal direction of one of the meander-like segments (8.1 - 8.8), thereby forming an inner profile (11) that protrudes into the passage (4).

2. The implantable compensating sleeve according to claim 1, **characterised in that** the at least one bulge (12.1 - 12.8) running in the longitudinal direction of a meander-like segment (8.1 - 8.8) extends over the two adjacent longitudinal arms (9.1 - 9.2, 9.3 - 9.4, 9.5 - 9.6, 9.7 -9.8, 9.9 - 9.10, 9.11 - 9.12, 9.13 - 9.14 and 9.15 - 9.16) of the meander-like segment (8.1 - 8.8).

3. The implantable compensating sleeve according to claim 1 or 2, **characterised in that** bulges (12.1 - 12.8) provided on the inner surface (2) of the sheath body (2) facing the passage (4) form a circumferential inner profile undulating in a cross-section of the compensating sleeve and having wave peaks (13.1 - 13.8) and wave troughs (14.1 - 14.8).

4. The implantable compensating sleeve according to claim 3, **characterised in that** one wave peak (13.1 - 13.8) and/or one wave trough (14.1 - 14.8) of the undulating inner profile (11) extends over two adjacent longitudinal arms (9.1 - 9.16) of a meander-like segment (8).

5. The implantable compensating sleeve according to claim 3 or 4, **characterised in that** the slits (7.1 - 7.16) running in the longitudinal direction of the sleeve body (3) are provided in alternation on the bottom of a wave trough (14.1 - 14.8) and on a crest of a wave peak (13.1 - 13.8).

6. The implantable compensating sleeve according to any one of claims 3 to 5, **characterised in that**
the undulating inner profile (11) is formed axially symmetrical with respect to the longitudinal axis of the compensating sleeve (1).

7. The implantable compensating sleeve according to any one of claims 1 to 6, **characterised in that**
the outer side (15) of the sheath body (3) formed by the outer surface of the longitudinal arms (7.1 - 7.16) of a meander-like segment (8) is formed unprofiled.

8. The implantable compensating sleeve according to any one of claims 1 to 7, **characterised in that**
the inner profile (11) is formed complementary to a groove profile (16) in an elongate implant portion of the first implant (2).

9. The implantable compensating sleeve according to any one of claims 1 to 8, **characterised in that**
the compensating sleeve (1) is made of a surgical steel, titanium, or a titanium alloy.

10. The implantable compensating sleeve according to any one of claims 1 to 8, **characterised in that**
the compensating sleeve (1) is formed from a biocompatible plastics material, in particular PEEK.

## Revendications

1. Manchette de compensation implantable (1) pour une endoprothèse, destinée à être posée entre un tronçon d'implant longitudinal d'un premier implant (2) et un deuxième implant entourant le tronçon d'implant longitudinal du premier implant,
la manchette de compensation (1) étant conçue sous la forme d'une douille pourvue d'un corps de douille (3) et d'un passage (4) traversant de l'extrémité proximale vers l'extrémité distale du corps de douille pour recevoir le tronçon d'implant longitudinal du premier implant (2),
le corps de douille (3) étant divisé par des fentes (7.1 à 7.16) entaillant le corps de douille (3) en direction longitudinale et partant en alternance du bord distal (5) et du bord proximal (6) du corps de douille en des segments (8.1 à 8.8) en forme de méandres, dotés chacun de deux bras longitudinaux (9.1 à 9.16), et
au moins dans un tronçon partiel, le corps de douille (3) étant étirable en direction radiale,
sur la surface interne (10) du corps de douille (3) qui fait face au passage (4) étant aménagé en formant un profilé interne (11) saillant à l'intérieur du passage (4) au moins un bombement (12.1 à 12.8) s'écoulant dans la direction longitudinale d'un segment (8.1 à 8.8) en forme de méandre.

2. Manchette de compensation implantable selon la revendication 1, **caractérisée en ce que**
l'au moins un bombement (12.1 à 12.8) s'écoulant dans la direction longitudinale d'un segment (8.1 à 8.8) en forme de méandre s'étend sur les deux bras longitudinaux (9.1 à 9.2, 9.3 à 9.4, 9.5 à 9.6, 9.7 à 9.8, 9.9 à 9.10, 9.11 à 9.12, 9.13 à 9.14 et 9.15 à 9.16) voisins du segment (8.1 à 8.8) en forme de méandre.

3. Manchette de compensation implantable selon la revendication 1 ou 2, **caractérisée en ce que**
des bombements (12.1 à 12.8) aménagés sur la surface interne (2) du corps de douille (2) qui fait face au passage (4) forment un profilé interne périphérique et ondulé dans la section transversale de la manchette de compensation, avec des crêtes d'ondes (13.1 à 13.8) et des creux d'ondes (14.1 à 14.8).

4. Manchette de compensation implantable selon la revendication 3, **caractérisée en ce qu'**une crête d'onde (13.1 à 13.8) et/ou un creux d'onde (14.1 à 14.8) du profilé interne (11) ondulé s'étend respectivement sur deux bras longitudinaux (9.1 à 9.16) voisins d'un segment (8) en forme de méandres.

5. Manchette de compensation implantable selon la revendication 3 ou 4, **caractérisée en ce que**
les fentes (7.1 à 7.16) s'écoulant dans la direction longitudinale du corps de douille (3) sont aménagées en alternance sur le fond inférieur d'un creux d'onde (14.1 à 14.8) et ou sur un sommet d'onde d'une crête d'onde (13.1 à 13.8).

6. Manchette de compensation implantable selon l'une quelconque des revendications 3 à 5, **caractérisée en ce que**
le profilé interne (11) ondulé est conçu en symétrie axiale de l'axe longitudinal de la manchette de compensation (1).

7. Manchette de compensation implantable selon l'une quelconque des revendications 1 à 6, **caractérisée en ce que**
la face extérieure (15) du corps de douille (3) formée par la surface extérieure des bras longitudinaux (7.1 à 7.16) d'un segment (8) en forme de méandre est conçue sans profilage.

8. Manchette de compensation implantable selon l'une quelconque des revendications 1 à 7, **caractérisée en ce que**
le profilé interne (11) est conçu en complémentarité d'un profilé cannelé (16) dans un tronçon longitudinal d'implant du premier implant (2).

9. Manchette de compensation implantable selon l'une quelconque des revendications 1 à 8, **caractérisée en ce que**
la manchette de compensation (1) est formée en un acier chirurgical, en titane ou en un alliage de titane.

10. Manchette de compensation implantable selon l'une quelconque des revendications 1 à 8, **caractérisée en ce que**
la manchette de compensation (1) est formée en une matière plastique biocompatible, notamment en PEEK.
